# EUROPEAN PATENT SPECIFICATION

(11) **EP 0 790 063 B1**
(45) Date of publication and mention of the grant of the patent: **02.10.2002**
(21) Application number: 97101753.8
(22) Date of filing: 05.02.1997
(51) Int. Cl.: A61M 5/00

(54) **A modular rack system for use in packaging and handling syringe barrels**
Modulares Ständersystem zum Verpacken und Handhaben von Spritzenzylindern
Système de support modulaire pour l'emballage et la manutention des corps de seringue

(30) Priority: 08.02.1996 US 598445
(43) Date of publication of application: 20.08.1997
(73) Proprietor: Becton Dickinson and Company, Franklin Lakes, New Jersey 07417-1880 (US)
(72) Inventor: Heyman, Peter W., County of Union, New Jersey (US); Francavilla, Frank, County of Sussex, New Jersey (US)
(74) Representative: Quinterno, Giuseppe

(56) References cited:
- WO-A-92/04255
- WO-A-94/14484

## Description

### 1. Field of the Invention

This invention relates to a system for packaging and handling elongate objects, particularly syringe barrels, and more particularly, to a rack system for packaging and handling syringe barrels to facilitate the filling of syringe barrels along a drug filling line, of the kind defined in the preamble of Claim 1.

### 2. Background

WO-A-94 14484 discloses a rack system of the above defined kind comprising a plurality of comb-shaped racks. Each rack has a plurality of fingers connected at one end by a spine member. The fingers thereby define parallel support channels open at one end. Elongate objects, such as syringe barrels, are located in the channels with their proximal end flanges resting on the fingers. The racks are usable both for packaging and for processing a quantity of syringes along a processing line. Support cradles are associated to the racks, however for supporting them only during processing of the syringes.

As is well known in the art, syringes are medical delivery devices capable of being filled with a quantity of medicament for delivery to a patient. Syringes typically include a number of distinct components, including a relatively elongate cylindrical barrel configured to receive the medicament. The barrel has a distal end mated to a piercing element such as a pointed needle cannula or a blunt ended cannula, and an open proximal end through which a stopper is inserted in sliding, fluid-tight relation with the inside wall of the barrel. A plunger rod is mated to the stopper, such that the user can urge the stopper distally to deliver medicament via the piercing element or urge it proximally to aspirate fluids into the barrel. A flange can be provided around the barrel at the proximal end to assist in the users manipulation of the syringe.

Syringes can be provided in an empty state, intended to be filled with medicament by the end user at or near the time an injection is desired. That is to say, a user has to himself fill the syringe from a bulk source of medicament, such as a vial. However, it is increasingly common that pharmaceutical manufacturers "pre-fill" syringes with a desired.quantity of medicament prior to shipping the medicament to an end user. Such syringes, known as "pre-fillable" or "pre-filled" syringes, are supplied by the syringe manufacturer to the pharmaceutical manufacturer in bulk quantities. The syringes are thereafter filled by the pharmaceutical manufacturer with a desired quantity of medicament and shipped to the end user in a ready-to-inject state.

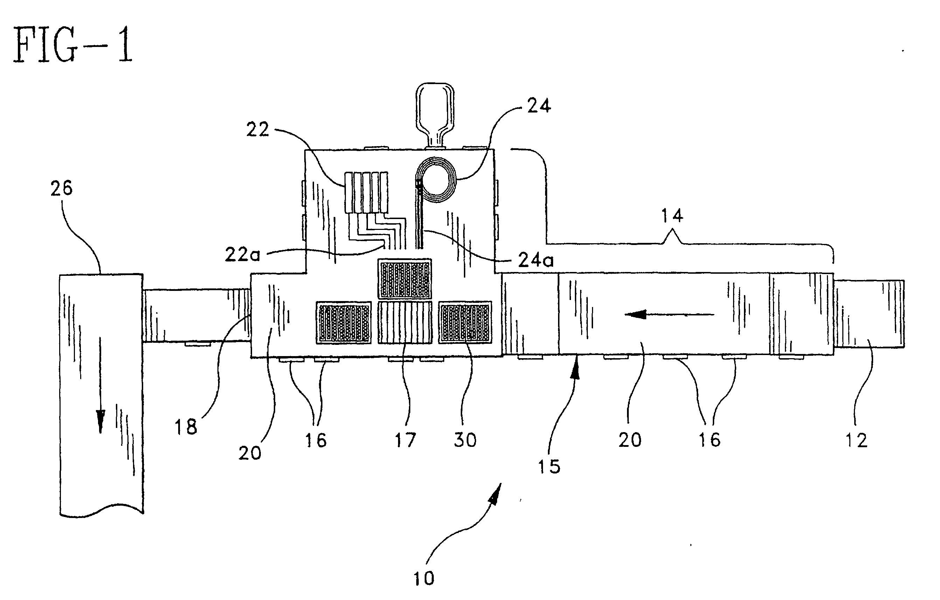

In order that the pre-filled syringe is supplied to the end user in a sterile state, measures have to be taken to ensure that the sterility of the empty syringes supplied by the syringe manufacturer, and afterwards, that the pharmaceutical manufacturer process and otherwise fill the syringes under conditions of sterility. One practice of pharmaceutical manufacturers is to process and otherwise fill the syringes in so called "clean-room" environments. However, Figure 1 depicts one current design of a syringe filling line 10, largely enclosed within a barrier isolator 14. Barrier isolator 14 is typically enclosed and pressurized with HEPA-filtered air to maintain a sterile processing environment. As the skilled artisan will appreciate, barrier isolators are increasingly common and have been supplanting clean-room environments for syringe filling operations. Filling line 10 includes a syringe loading station 12 where syringes 30 in packaged form are unloaded in bulk state for transport through barrier isolator 14 via conveyor 20. The barrier isolator also includes one or more glove-type access ports 16. Owing to the size of barrier isolator 14, numerous access ports are typically provided and are located on most sides of the barrier isolator. Syringes 30 pass through a decontamination area 15 for certain surface decontamination treatments, followed by an operator's manual removal of packaging materials via the glove-type access ports. The syringes may also manipulated in preparation of the filling process. After the syringes are unpacked, they are placed on a fixture 17 for transport to filling (22) and stoppering (24) stations within the barrier isolator. Here, syringes 30 may be filled, in quantity, from a sterile source of medicament using one or more filling nozzles 22a, and stoppered in quantity using one or more stoppering heads 24a. Once filled and stoppered, syringes 30 are manually removed from fixture 17 for transport by conveyor 20 out of barrier isolator 14. The syringes exit the barrier isolator at an opening 18, and thereafter proceed to a plunger rod station 26 where plunger rods (not shown) are affixed.

One drawback of current filling lines 10 is the relatively large space for the operator to manipulate syringes 30, and their associated packaging, through the enclosed barrier isolator 14. Owing to the relatively high speeds at which most filling lines operate, it would be desirable to minimize the quantity of and space occupied by the packaging associated with syringes 30, in an effort to reduce the size of the barrier isolator.

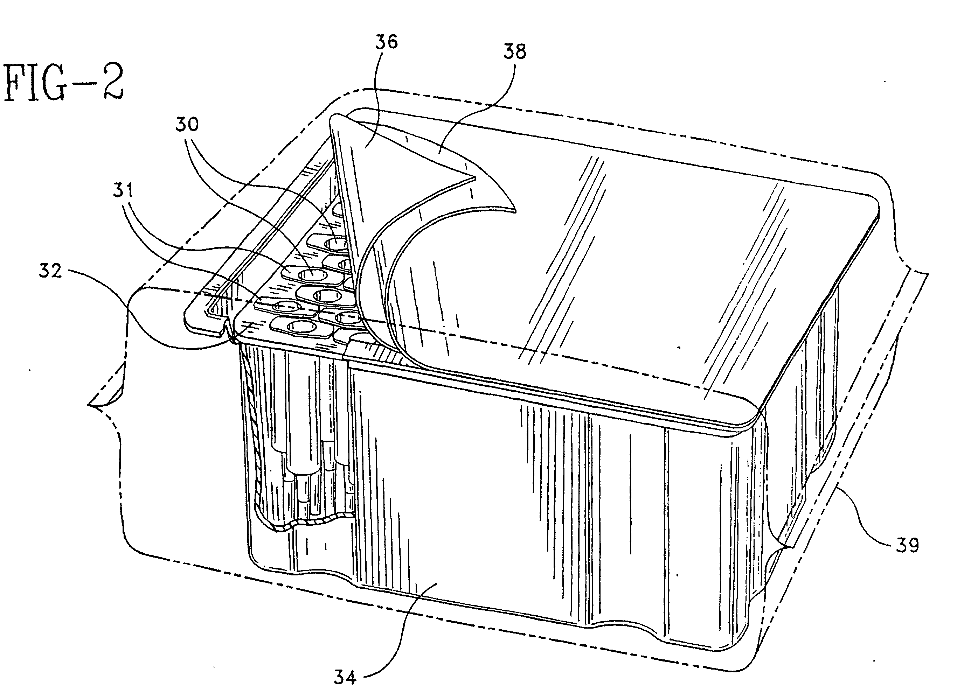

One current approach to syringe packaging, depicted in Figure 2, is the HYPAK® SCF® system sold by Becton, Dickinson and Company of Franklin Lakes, New Jersey, USA. Syringes 30 are individually inserted in separate compartments (not shown) defined by a rigid plastic nest 32, with the respective flange 31 of each syringe 30 resting against nest 32. Syringe 30, and in particular the relatively elongate barrel 33 of the syringes, are held relatively loosely by each of the separate compartments. The nest 32 is itself supported by a lip (not shown) within plastic tub 34. A layer of foam material 36 may be placed over the syringes, and thereafter the tub 34 is sealed by a Tyvek layer 38. The Tyvek-sealed tub 34 is further enclosed by a surrounding plastic bag 39.

In preparing the syringes for filling, an operator must at least remove plastic bag 39, Tyvek layer 38, foam material 36, and usually tub 34. The materials tend to accumulate within the enclosed barrier isolator 14 and require periodic removal by the operator in a manner which avoids compromising sterile conditions within the barrier isolator. In view of the high speeds at which syringes are processed, and to make the operator's task easier, it would be desirable to minimize the amount of packaging material which accumulates within barrier isolator 14. Filled syringes 30 are thereafter conveyed as a block on conveyer 20. The size of plastic tub 34 requires a commensurately large opening 18 at the interface between barrier isolator 14 and the remainder of filling line 10. Also, the large number of access ports 16 required increases the probability of compromising sterility in the barrier isolator because of leaks through the ports. iIn view of the need to maintain sterile conditions within the barrier isolator and the pressurization associated with the enclosed barrier isolator, it would be desirable to minimize the number of access ports 16 and the size of opening 18. Furthermore, while most filling lines 10 feature equipment along filling conveyer 20 designed to handle the block form packaging described above, the filling speed for the line is somewhat limited, owing to the need to duplicate filling head and conveying structures in order to process multiple tubs at a time. It would be useful to package the syringes in a way allowing an operator to process the syringes either in block-form, or in sequential batches to optimize or otherwise simplify the designs of high-speed filling lines. Also, by designing packaging to optimize sequential processing, there is the possibility of reducing the dimensions of barrier isolator 14, thereby optimizing sterility maintenance and particulate control through improved air handling within the isolator. With the exisiting packaging, syringes 30 are held somewhat loosely within their individual compartments, there is a chance that the syringes can become misaligned during filling and stoppering operations. Further, because syringes 30 are largely shielded from the operator's view by nest 32 and tub 34, visual inspection of the syringes is difficult.

### 3. Summary of the Invention

The invention achieves the object of improving the rack system of the initially-defined-kind by the features defined in Claim 1. It will be appreciated by the skilled artisan that the features and advantages of the present invention are not limited to handling syringe barrels. Rather, the features and the advantages of the present invention are readily applicable to processing and handling any elongate object along a processing line, particularly where flexibility is desired in handling or processing the elongate objects either as a block entity or in a sequential manner.

The syringe rack in accordance with the present invention features two or more vertical supports attached to a base for supporting the syringe rack on a work surface. The syringe rack includes structure for releasably but firmly supporting one or more syringes in the rack. A plurality of barrel clips are secured to a bar attached between the vertical supports of the syringe rack. The barrel clips are positioned to releasably grasp the syringe barrels along their length and to vertically orient the syringe barrels respective of the work surface.

Structure is provided to secure the flanges associated with each of the syringes held by the rack. In one configuration, the flange support includes a pair of plates affixed to an upper extremity of the vertical supports and spaced apart from one another to define a pocket for receiving the flanges. A plurality of dividers are provided between the plates so as to divide the pocket into a plurality of compartments. Each of the compartments is configured to receive a respective flange of a syringe. A series of cut-outs are provided on each of the plates. The cut-outs on the upper plate are located so as to rest over the open proximal end of each of the syringe barrels held by the rack, while the cut-outs on the lower plate are located to accommodate entry of the syringe barrel adjacent the flange. The top plate is relatively flat, allowing the racks to be stacked one on top of the other for ease of packaging and handling.

In a further embodiment, the structure for securing the flanges of the syringes includes a top plate fixed to an upper extremity of the vertical supports, and a side plate secured between the vertical supports in orthogonal relation to the top plate. Similar to the prior embodiments, the top plate includes a plurality of dividers to divide the top plate into a plurality of individual compartments. A plurality of cut-outs are also provided on the top plate and disposed over the open proximal end of each of the syringe barrels held by the rack. The side plate includes one or more slits horizontally provided through the side plate. The slits are sized to accept a portion of a respective flange introduced into an individual compartment.

Various structure may be incorporated to permit one or more of the syringe racks to be releasably secured to one another in order to form a block configuration of syringe racks. For instance, one or more resilient pins can be formed on the rack. The pins are releasably insertable into respective depressions formed in another of the racks so as to releasably secure the racks to one another. Alternately, the syringe racks may be releasably secured to one another by variations of tongue and groove structure provided on the bases of the racks. The overall configuration permits the racks to be attached to one another into a block configuration for packaging or handling purposes. If desired, the operator can process the syringes held by the racks in their block configuration. Alternately, the operator can manually or mechanically detach individual syringe racks from the block configuration along the processing line, allowing sequential processing of the syringes held by the individual racks.

Similarly, individual syringe racks can be formed or otherwise attached to one another by flexible couplings formed along the base or the vertical supports. The flexible couplings allow the syringe racks to assume an accordion-like block configuration for packaging or handling. If desired, the individual syringe racks can be flexibly separated from their accordion-like block configuration such that the individual racks are positioned into a line formation for sequential processing.

The syringe rack in accordance with the present invention minimizes the amount of packaging material necessary. The size of the syringe rack minimizes the size of the opening necessary between a pressurized barrier isolator and the remainder of the drug filling line. The syringes can be processed in either block form or in a sequential series of operations, as need or desire dictate. Moreover, owing to the open architecture of the syringe rack, the operator can easily perform any visual tasks associated with the drug filling operation.

### 4. Brief Description of the Drawings

The invention will now be described in detail by way of reference to the following drawings, wherein:
Figure 1 is a top plan view of one syringe filling line currently in use;
Figure 2 is a perspective view of a prior art approach to packaging syringes for syringe filling lines;
Figure 3 a front perspective view a first embodiment of a syringe rack in accordance with the present invention;
Figure 4 is a front elevational view of the embodiment depicted in Figure 3;
Figure 5 is a top plan view of the embodiment depicted in Figure 3;
Figure 6 is a side elevation view of the embodiment depicted in Figure 3,
Figure 7 is a rear elevational view of the embodiment depicted in Figure 3,
Figure 8 is a front perspective view of another embodiment of a syringe rack in accordance with the present invention;
Figure 9 is a front perspective view of a further embodiment of a syringe rack in accordance with the present invention;
Figure 10 is a top plan view of the embodiment depicted in Figure 9;
Figure 11 is a front elevational view of a syringe rack in accordance with the present invention depicting the use of snap pins and orifices to detachably connect one or more syringe racks together;
Figure 12 is a rear elevational view of the syringe rack depicted in Figure 11;
Figure 13 is a side elevational view of a syringe rack in accordance with the present invention depicting the use of a tongue and groove arrangement to detachably connect one or more syringe racks together;
Figures 14a and 14b are side elevational views of a syringe rack in accordance with the present invention depicting variations of a tongue and groove arrangement to detachably connect one or more syringe racks together;
Figure 15 is a top plan view of another embodiment of a syringe rack in accordance with the present invention, wherein the syringe racks are attached to one another in an accordion arrangement,
Figure 16 is a top plan view of a proposed syringe filling line taking advantage of the reduced size packaging presented by a syringe rack in accordance with the present invention; and
Figure 17 is a schematic view of one way to process a syringe rack in accordance with the present invention for use in a typical syringe filling line.

### 5. Detailed Description of the Preferred Embodiments

A convention employed in this patent application is that the term "distal" refers to a direction furthest from a practitioner, whereas the term "proximal" refers to a direction closest to the practitioner.

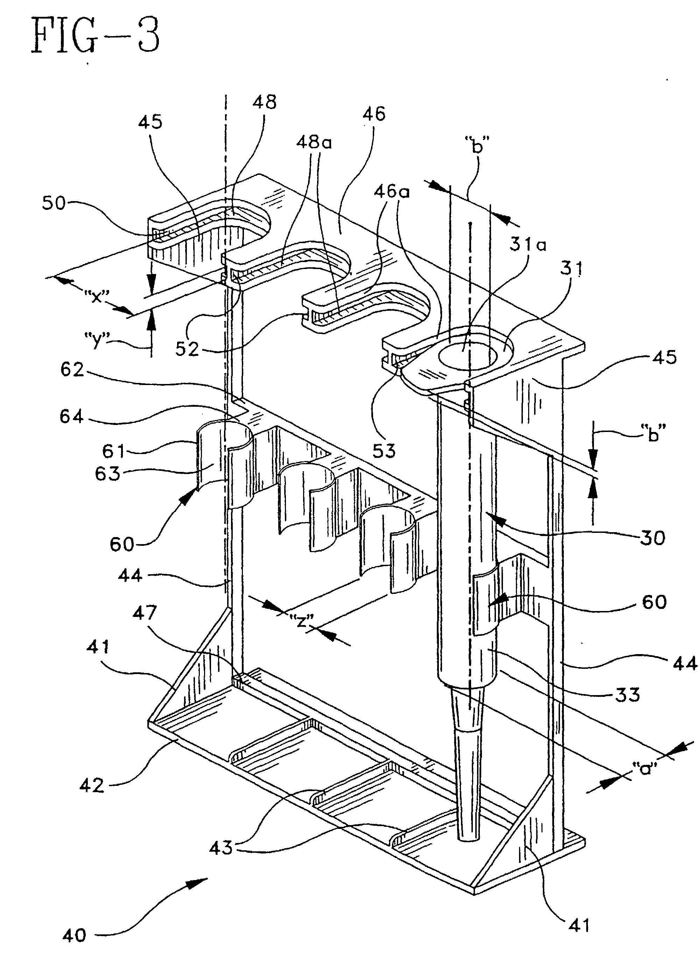

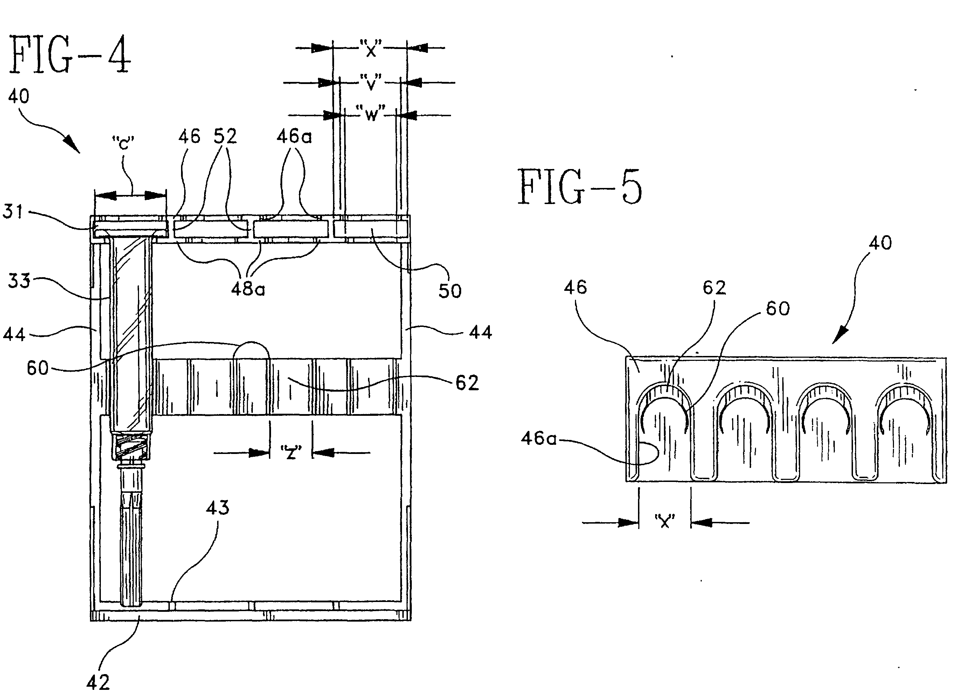

Turning now to the drawings, wherein like numerals denote like components, Figures 3-7 depict one embodiment 40 of a syringe rack in accordance with the present invention. Syringe rack 40 features two or more vertical supports 44 affixed at their lower extremity to a relatively flat base 42. The number of vertical supports 44 can be chosen according to the width of syringe rack 40 or any necessary structural support required of syringe rack 40.To enhance structural integrity, support wedges 41 can be affixed between base 42 and respective vertical supports 44. A strengthening rib 47 can be provided on base 42 between vertical supports 44 for the same purpose. Also, one or more riblets 43 can be provided on base 42, both to lend structural integrity to syringe rack 40, and to divide base 42 into a number of sections, each of which will be associated with the retention of a respective syringe 30.

An advantage of syringe rack 40 vis-a-vis prior art approaches is the open architecture presented by the rack, permitting ready visual observation of the syringes, either by human operators or by camera-based systems, along the processing line. An additional advantage of syringe rack 40 in accordance with the present invention is the ability to facilitate removal of, and subsequent replacement of, syringes 30 from and to the rack at any point in the filling process. For instance, it may be necessary or desirable to conduct in-process weight checking of syringes 30 during the filling operation. This would require the removal of syringes 30 from their packaging. The construction of syringe rack 30 in accordance with the present invention facilitates easy removal and replacement of the syringes from or to the rack, while still preventing unwanted movement or jostling, which could lead to handling or misalignment problems along the processing line.

To this end, Figures 3-7 depict structure for firmly but releasably securing syringes 30 within each of syringe racks 40. The structure includes means both for releasably securing each of flanges 31 associated with the syringes, together with structure for releasably securing each of syringe barrels 33. Referring principally to Figures 3-6, one or more resilient syringe clips 60 can be provided for releasable retention of individual syringe barrels 33. In the configuration depicted, syringe clips 60 include a partial circumferential wall 61 secured to a bar 62, itself attached to vertical supports 44. Each of partial circumferential walls 61 is secured to the bar via a series of individual necks 64. Each of partial circumferential walls 61 includes an opening 63 having a width "z" slightly less than the outside diameter "a" of syringe barrel 33. In this manner, syringe barrel 33 can be inserted into syringe clips 60, such that partial circumferential wall 61 will resiliently expand about the syringe barrel so as securely but releasably retain it within the syringe clip. It will be evident to the skilled artisan that the size, placement, or number of syringe clips 60 can vary according to the dimensions of or number of syringes 30 intended to be retained by syringe rack 40. Moreover, it will be evident to the skilled artisan that syringe clips 60 may be affixed to syringe rack 40 in alternate manners. For instance, the structure associated with syringe clips 60 or the manner for retaining them to syringe rack 40 can be designed so as to minimize the quantity of material necessary to produce them.

Syringe rack 40 also features structure for securely but releasably retaining each of flanges 31 associated with individual syringes 30. Referring to Figures 3-7, in one embodiment, a pair of top and bottom plates 46 and 48, respectively, are affixed at an upper extremity of vertical supports 44. Top plate 46 is relatively flat, allowing one or more racks 40 to be stacked one on top of the other, the base of a syringe rack 40 resting on the top plate of another rack. If desired, side wedges 45 can be incorporated between the vertical supports and the plates so as to enhance structural integrity of the construction. Top and bottom plates 46 and 48 are spaced apart from one another to define between them a pocket 50 dimensioned to receive flanges 31. A plurality of dividers 52 are disposed between the top and bottom plates so as to divide pocket 50 into a series of individual compartments 53 sized to individually receive a respective flange 31. Pocket 50 can display a width "y" equal to if not slightly greater than the thickness "b" of syringe flange 31 in order to minimize play or jostling of the syringe flanges while retained by pocket 50. Also, each of compartments 53 features a width "x", measured between adjacent dividers 52, that is equal to if not slightly greater than a minimum width "c" displayed by syringe flange 31. Thus, any play or jostling between syringe flange 31 and compartment 53 is minimized, further contributing to alignment of the syringe as it proceeds along filling line 10

Each of top plate 46 and bottom plate 48 feature respective cut-outs 46a and 48a. Cutouts 46a are disposed over compartments 53 so as to provide ready access to the open proximal ends 31a of the syringe barrels to facilitate filling and stoppering operations. Each of cut-outs 48a are positioned on bottom plate 48 to permit entry of a proximal section of syringe barrel 33 located adjacent flange 31. Width "v" of cut-out 46a is preferably at least as wide as internal diameter "b" of syringe barrel 33 to facilitate access to the syringe barrel for filling and stoppering operations. Width "w" of cut-out 48a is preferably equal to if not slightly greater than outside diameter "a" of syringe barrel 33 so as to facilitate entry of the proximal section of the syringe barrel adjacent flange 31, as previously described.

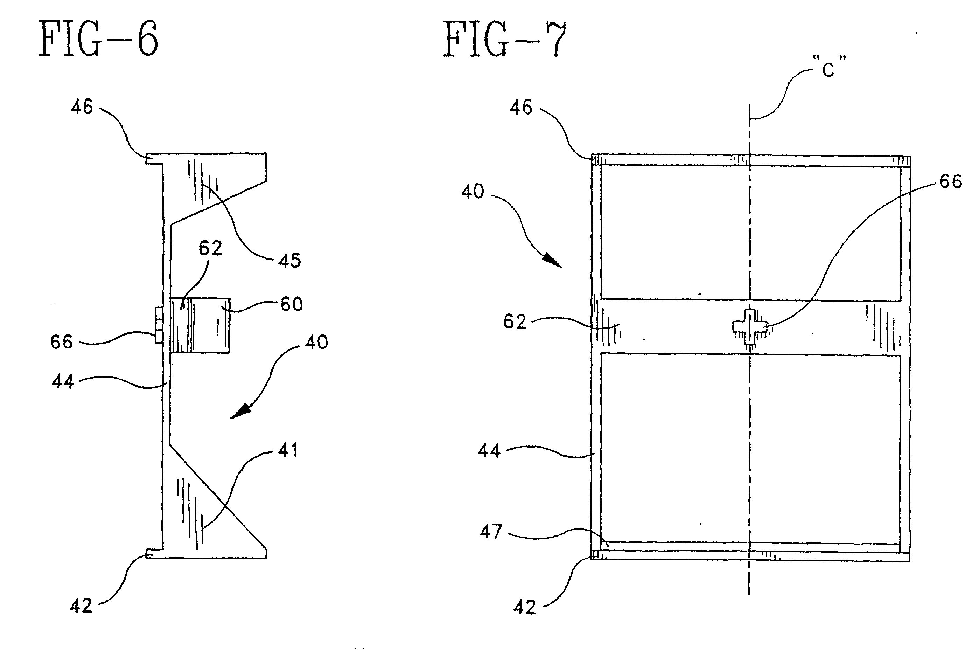

Structure can be provided to facilitate manual or mechanical alignment of syringe rack 40 as it proceeds along the filling line. For instance, referring to Figure 7, an indexing structure 66 can be provided on support bar 62 along the center line "c" defined by syringe rack 40. Indexing structure 66 can be formed as a cut-out in, or as a protrusion emanating from, bar 62. Here, indexing structure 66 is shown as a cross-shaped protrusion emanating from the bar. The processing machinery associated with filling line 10 can thus be configured or otherwise structured to align syringe rack 40 from indexing structure 66, so as to preserve alignment of syringe barrels 30 along the filling line. By placing the indexing structure along the center line of the rack, the risk of mis-alignment because of tolerance or dimensional deviations is minimized.

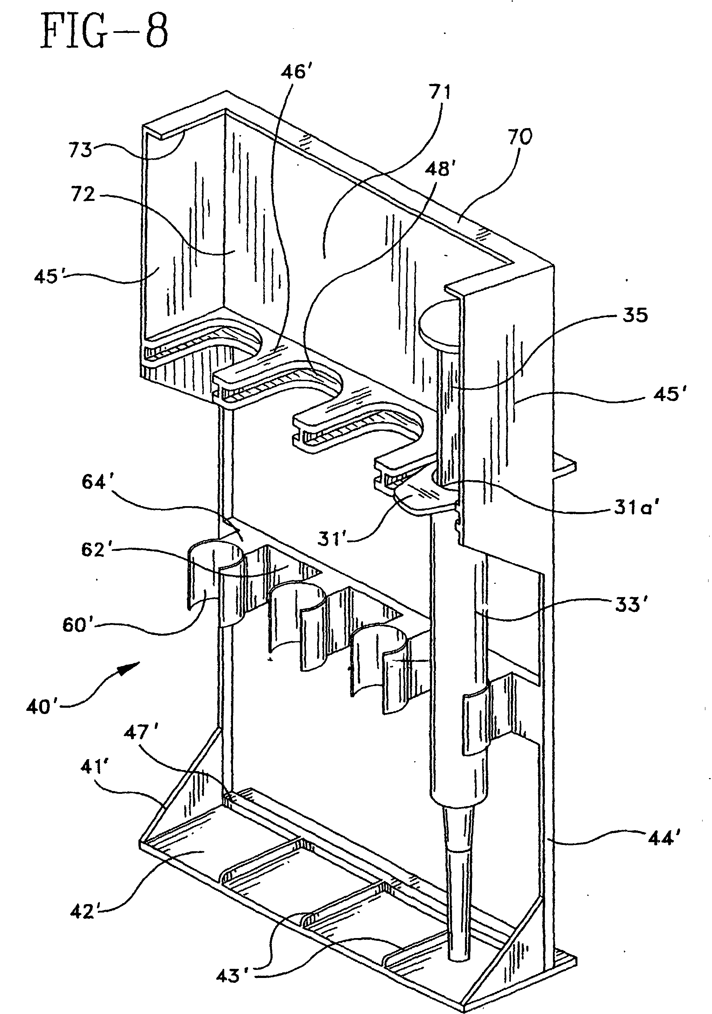

Figure 8 depicts an alternate embodiment 40' of a syringe rack 40 in accordance with the present invention. Components common with embodiment 40 depicted in Figures 3-7 are designated by like numerals, except they are denoted by an apostrophe ('). Syringe rack 40' is distinguished from syringe rack 40 by the provision of an extension 72 configured to retain a component, such as a plunger rod 35, that protrudes from open proximal end 31a' of the syringe. Side wedges 45' extend vertically from top plate 46', with a shelf 70 affixed to an upper extremity of side wedges 45'. Shelf 70 is distinguished by a perimeter 73 providing access to extension 72 that is defined between top plate 46' and shelf 70. As in the prior embodiment, shelf 70 is relatively flat, allowing syringe racks 40' to be stacked one on top of the other. If desired, a back wall 71 can be secured between side wedges 45', top plate 46', and shelf 70, so as to enclose extension 72 on five sides. However, rear wall 71 is not necessary and can be omitted to facilitate visual observation of the syringes held by syringe rack 40'.

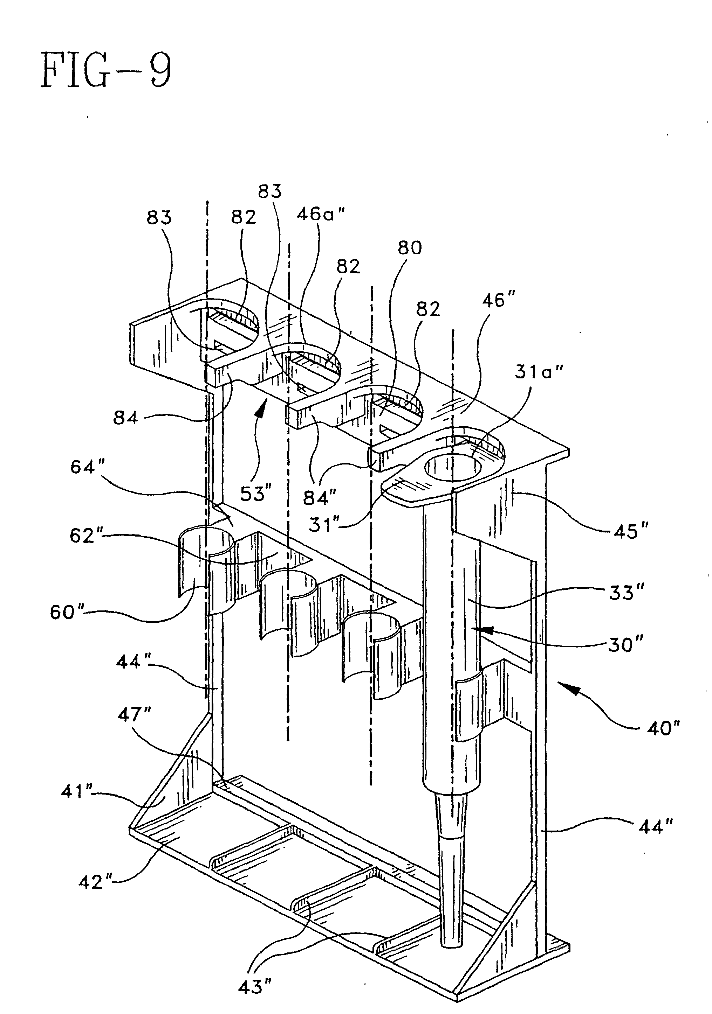

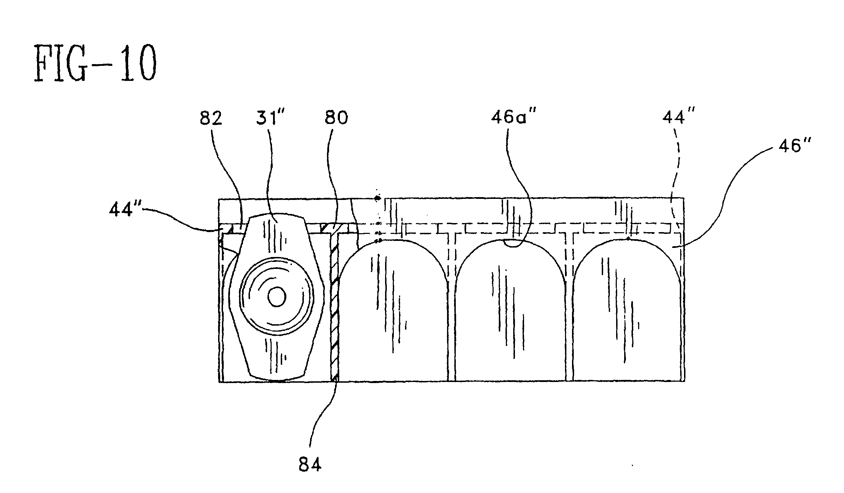

Figures 9 and 10 depict a third embodiment 40" of a syringe rack in accordance with the present invention. As before, like numerals denote like components of the syringe rack as previously described, apart from the provision of a double-apostrophe (") Here, alternate structure is provided to secure flanges 31" the rack. A top plate 46" is secured to an upper extremity of side wedges 45". One or more dividers 84 emanate from a bottom surface of top plate 46" so as to divide top plate 46 into a series of compartments 53" dimensioned to receive a respective syringe flange 31". Top plate 46" also features a plurality of cut-outs 46a" dimensioned and configured on top plate 46" so as to provide access to each of the open proximal ends 31a" of syringes 30 held by syringe rack 40".

In lieu of forming a flange-receiving pocket, a side wall 80 is secured to vertical supports 44", in orthogonal relation to top plate 46", in a manner extending across each of compartments 53" of the top plate. One or more flange-receiving slits are provided through side wall 80 configured to secure a portion of flange 31" introduced into compartment 53". As herein shown, side wall 80 features, within the area encompassed by each of compartments 53", a pair of slits 82, 83. Each of slits 82 and 83 are dimensioned and otherwise placed on side wall 80 to receive flanges 31" of varying sizes or height of placement relative to syringe barrel 33". For instance, slit 82 can be placed on side wall 80 at a location above slit 83, so that syringe rack 40" can accommodate the insertion of flanges 31" associated with differing heights (or sizes) of syringes 30". It will be evident to the skilled artisan that the depth, thickness, or width of slits 82, 83 can be chosen so as to minimize movement or play of flange 31" retained by the slit. Alternately, in lieu of slits provided through side wall 80, varying recesses, pockets, or like structure can be provided on side wall 80 for the same purpose.

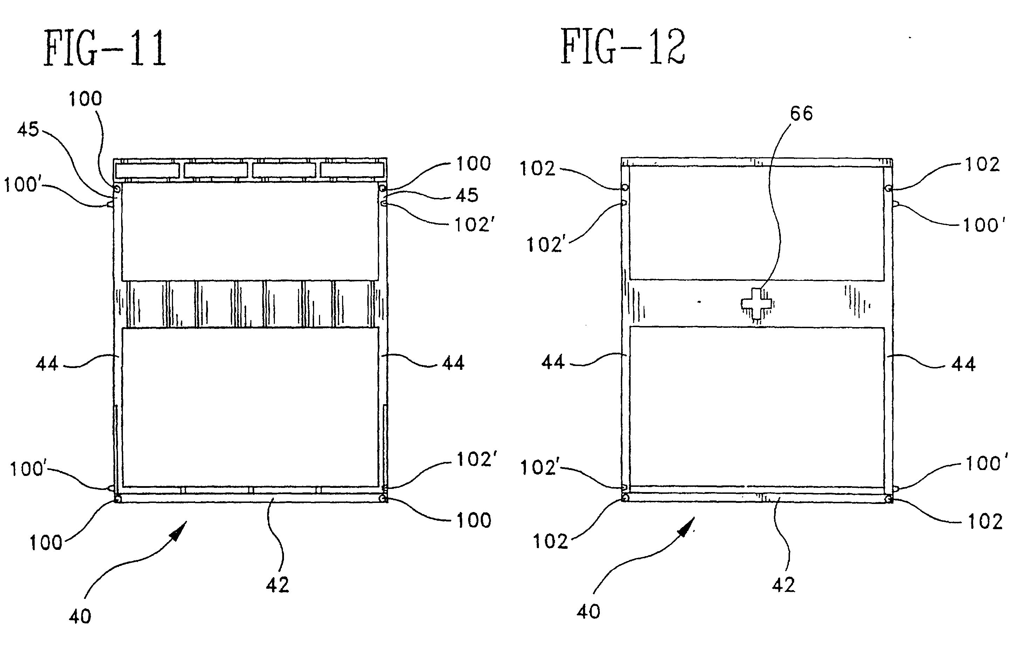

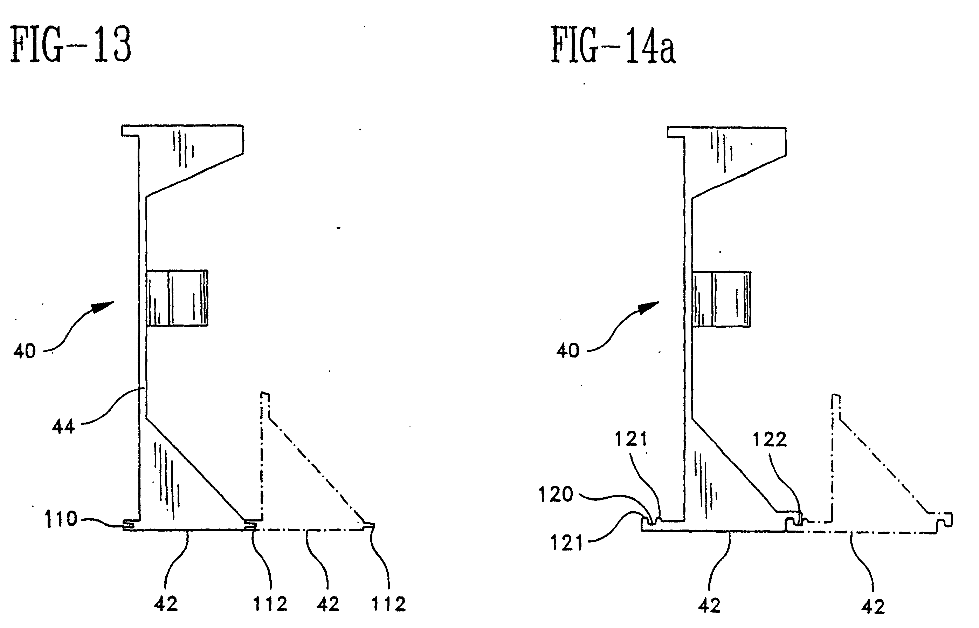

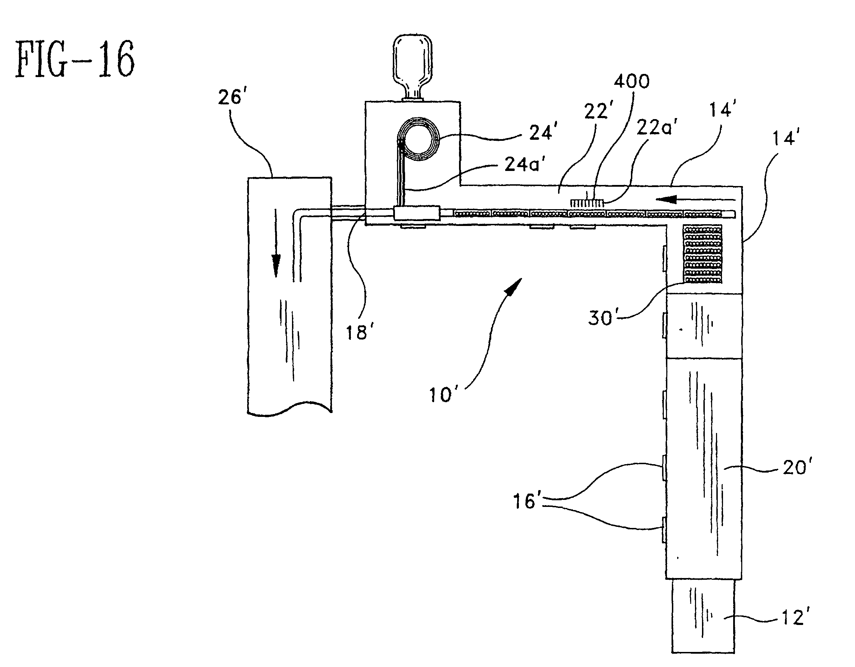

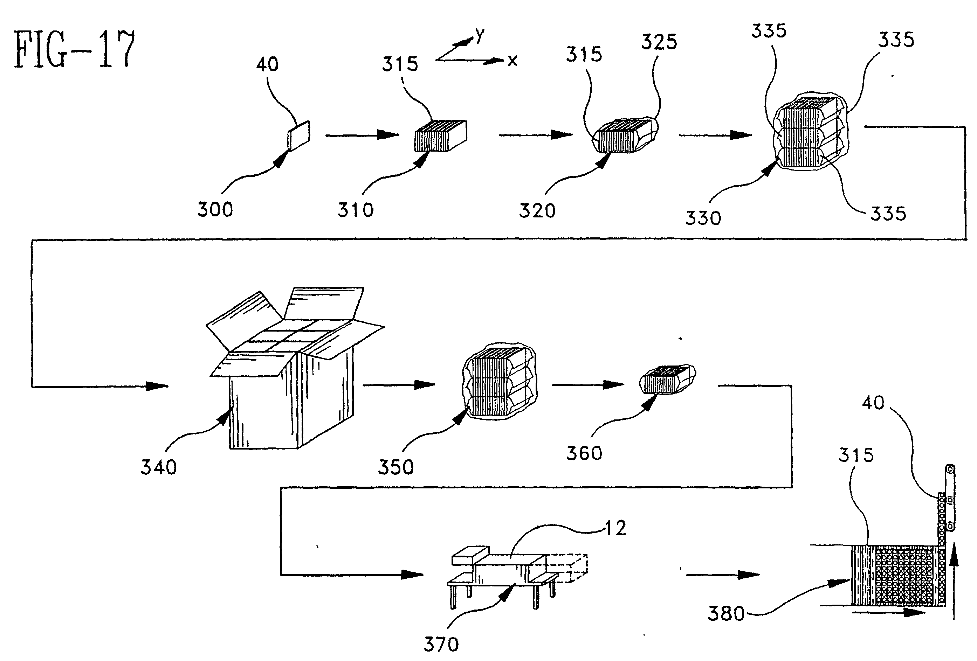

An advantage of the syringe rack in accordance with the present invention over other packaging configurations known in the prior art is the ability to package and handle a quantity of syringes 30 either together as a block, or to separate a given quantity of syringes 30 from the block configuration such that discrete batches of syringes may be sequentially processed along the filling line (see Figure 16 and the ensuing discussion). Figures 11 and 12 depict one manner for configuring the syringe rack of the present invention for ready attachment to another of said syringe racks. As depicted, one or more pins 100 and one or more depressions 102 may be provided on the syringe rack 40. In the configuration depicted by Figures 11 and 12, four pins 100 are provided on a frontal surface of syringe rack 40, one of the pins 100 formed on each of side wedges 45 and one pin 100 formed on opposed ends of base 42. The four pins are configured to snap or otherwise engage in recesses 102 formed in complementary locations on a rear surface of syringe rack 40. Thus, a given syringe rack 40 can be "snap-fit" onto an adjoining syringe rack 40 such that the syringe racks can be nested together in facing relationship to form a block configuration. If desired, pins 100' can be provided on an outside portion of vertical support 44 for mating contact with recesses 102' formed in a complementary location on vertical support 44 of an adjoining syringe rack. Accordingly, syringe racks 40 can be nested in both the x and y coordinate directions (see Figure 17) to form a block configuration of syringe racks.

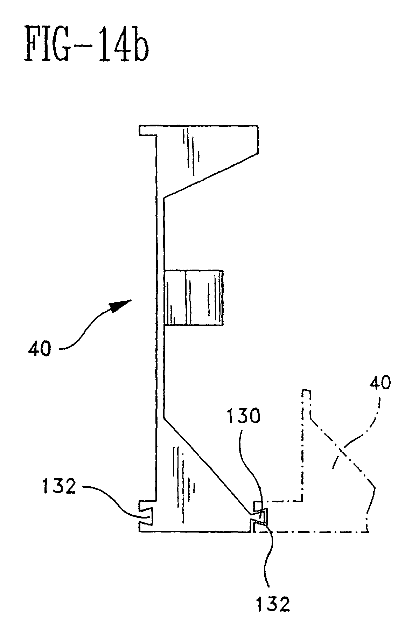

Alternate manners for nesting the syringe racks into a block configuration are depicted in Figures 13, 14a and 14b. Each of Figures 13, 14a and 14b illustrate manners for incorporating a tongue and groove structure to the bases of the syringe racks in order that they may be nested into a block configuration. In Figure 13, the front and rear edges of base 42 can be configured to include a horizontally-aligned groove 110 along one edge, and a horizontally-aligned tongue 112 along the other. The syringe racks can be nested together in block configuration, such that a tongue 112 of one syringe rack 40 is mated to a complementary groove 110 on an adjoining syringe rack. The dimensions and tolerances of the respective grooves and tongues 110, 112 can be dimensioned such that syringe racks 40 are firmly held in place while in the block configuration, but such that bases 42 are slidable relative to one another, permitting the separation of individual syringe racks 40 from the block configuration. If desired, the pins and recesses of the embodiment depicted in Figures 11 and 12 can be incorporated onto the sides of vertical supports 44 of a syringe rack in accordance with this embodiment (not here illustrated) The syringe racks will still be able to slide respective of one another for separation from the block configuration, with the pins and recesses preventing unwanted detachment until desired.

Figures 14a and 14b are a modification of the tongue and groove design depicted by Figure 13. In Figure 14a, an L-shaped tongue 122 is horizontally disposed along one of the frontal or rear edges of base 42. On the opposed edge of base 42, a U-shaped groove 120 is horizontally disposed. A pair of teeth 121 flank either side of U-shaped groove 120. An L-shaped tongue 122 of a given syringe rack 40 is "locked" between teeth 121 and U-shaped groove 120 of an adjoining syringe rack 40. Again, the dimensions and tolerances of the various components can be selected such that adjoining syringe racks are held in block configuration, permitting bases 42 to slide relative to one another to detach individual syringe racks from the block. In Figure 14b, a triangular tongue 130 formed along the base of one syringe rack mates with a complementary triangular groove 132 formed along the base of an adjoining syringe rack 40. This construction, commonly known as "dove-tail" construction in the carpentry arts, prevents the syringe racks from being pulled apart from one another; once the tongue is slid into the groove, the syringe racks are locked together. However, tongue 130 and groove 132 can be dimensioned so as to be slidable relative to one another to allow separation of the syringe racks by relative sliding motion between the bases.

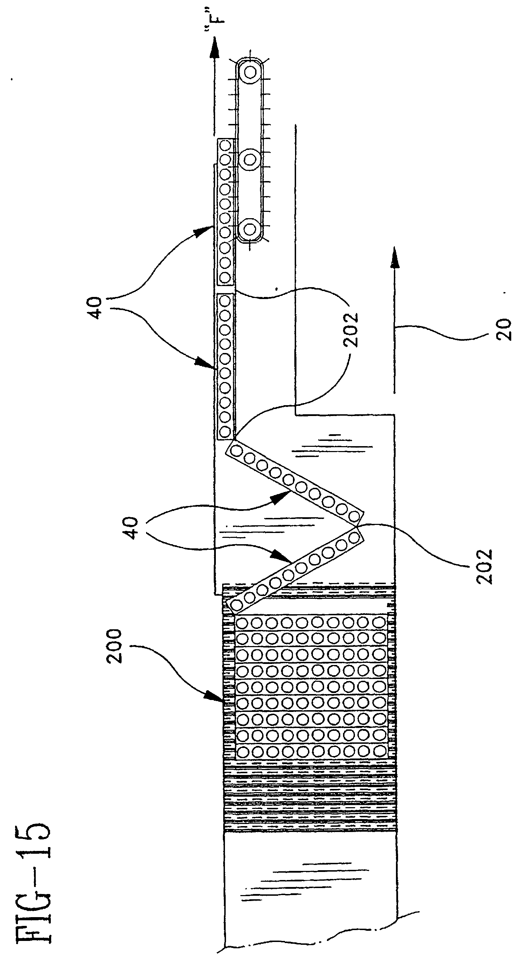

Figure 15 depicts an alternate way for securing individual syringe racks 40 into a block configuration 200 of such syringe racks. Here, each of syringe racks 40 are connected to one another by a flexible coupling 202. Flexible coupling 202 can be formed along the bases of vertical supports of the individual syringe racks, and can include, for instance, mechanical hinges, living hinges, or other types of flexible couplings known to the skilled artisan Here, syringe racks 40 are flexibly coupled to one another such that block configuration 200 assumes an "accordion" shape. While syringe racks 40 are not directly detachable from one another when separated from block configuration 200, owing to flexible couplings 202, each of the individual syringe racks 40 can be flexibly separated from the block configuration, such that the individual syringe racks 40 are positioned into a line formation at a point along filling line 10. Here, it is schematically shown that syringe racks 40 are flexibly separated from block configuration 200 via a force "F" exerted upon the syringe racks by equipment associated with filling conveyer 20. However, it will be evident to the skilled artisan that other mechanical and manual means can be employed to flexibly de-couple individual syringe racks 40 from block configuration 200 at any point along filling line 10.

Figures 16 and 17 depict ways of employing a syringe rack 40 in accordance with the present invention in a filling operation. Figure 16 is a top plan view representation of a syringe filling line 10' optimized to take advantage of the reduced size presented by a syringe rack 40 in accordance with the present invention. Figure 17 schematically represents steps depicting packaging use of syringe rack 40 for use in a filling operation.

Referring to Figure 16, components common with the syringe filling line 10 of Figure 1 are noted by the same numeral apart from the designation of an apostrophe ('). Barrier isolator line 14' can be made to smaller dimensions, particularly in the area of filling station 22' and stoppering station 24', owing to the reduced size of the packaging. Because the syringe racks can be separated from their block configuration, individual syringes 30 may be sequentially filled at filling station 22', from a sterile source of medicament, via a so-called walking beam 400. Nozzles 22a' are affixed to walking beam 400, each nozzle 22a' oreiented to fill a separate syringe 30. Owing to the reduced size of the barrier isolator, the number of access ports 16' can be reduced, and they can be placed on only one side of the barrier isolator Overall, then, the probability of compromising sterility in barrier isolator 14' because of leakage through the access ports is reduced.

Referring to Figure 17, at step 300, an individual syringe rack 40 is manufactured and filled with a quantity of individual syringes 30. At step 310, multiple syringe racks 40 are nested together to form a block configuration 315 of the syringe racks. Step 310 illustrates that the individual syringe racks can be nested in both the x and y plane, as desired. At step 320, a block configuration 315 of syringe racks is prepared for shipment in the usual manner. For instance, block 315 can be subjected to certain asceptic and sterilization treatments. Here also, a sterile cover 325, such as a plastic cover, can be secured over block configuration 315. If desired, to facilitate shipment, step 330 illustrates that a plurality of block configurations 3 15 can be stacked one on top of the other, with an outer cover 335 applied to the stack. Block configurations 315 can be packed according to the usual procedures for shipment (step 340) and thereafter removed by the filling operator in an appropriate sequence of operations (steps 350 and 360), such that block configuration 315 is introduced at syringe loading station 12 of the filling line (step 370). Thereafter, any desired mechanical or manual manipulations of the syringe racks or their packaging can be performed along the filling line. As depicted by Figure 17, the actual packaging associated with block configuration 315 is minimal, owing to the small quantities of plastic covering 325 (or 335) employed. The syringes contained within each of syringe racks 40 can be processed together in the block configuration 315; however, if desired, step 380 illustrates that individual syringe racks 40 be separated from block configuration 315 in order that the syringes held by each of the syringe racks are sequentially processed along the filling line.

Numerous features of the present invention can be implemented as necessary by the skilled artisan. The syringe rack can be formed in a unitary manner, or various components can be formed separately and thereafter attached to one another. The syringe rack is easily moldable from thermoplastic materials, and if desired, the materials employed for the various components can be selected according to need or desire. For instance, the base and vertical supports can be formed of rigid thermoplastics to prevent bending, while the syringe clips can be formed from resilient thermoplastics to allow secure but releasable retention of the syringe barrels. The dimensions of the syringe rack can be formed so as to accomodate various sizes or numbers of syringes. Also, the locations of pins, depressions, grooves, tongues, or other structure associated with releasably retaining the racks to one another can be varied according to need or desire.

It will be seen that the syringe rack in accordance with the present invention provides a line operator with the flexibility to process syringes either in bulk, through the block configuration, or sequentially within each of the racks.. A minimal amount of packaging material is necessary, minimizing both the complexity of the filling line and the size of the barrier isolator. The open architecture of the syringe rack provides ready opportunity for human or camera-based visual inspection of the syringes through all phases of the filling operation. Because the syringes are securely held by each of the racks, jostling or play of the syringes along the filling line is largely avoided, helping to prevent misalignment difficulties during various stages, such as filling and stoppering.

It will be appreciated by those skilled in the art that further and additional forms of the invention may be devised without departing from the scope of the appended claims, the invention not being limited to the specific embodiments shown.

## Claims

1. A rack system for packaging and processing a quantity of elongate objects, particularly syringe barrels (33; 33'; 33") along a processing line (10; 10') comprising a plurality of racks (40; 40', 40") having means (48; 80-83) for releasably retaining in a vertical orientation one or more of said elongate objects; **characterised by** each of said racks (40; 40'; 40") having means (100, 102; 100'; 102'; 110, 112; 121,122; 130, 132; 202) for releasably or flexibly coupling said racks to one another to form a block configuration (200; 315) of said quantity of elongate objects retained by said racks, wherein said block configuration (200; 315) of said elongate objects is adapted to be processed as a unit along said processing line (10; 10'), and said racks (40; 40'; 40") are adapted to be de-coupled from one another or flexibly separated from said block configuration to form a plurality of batches or a line formation of said elongate objects for sequential processing along said processing line (10; 10').

2. A rack system according to Claim 1, further comprising a protective cover (325; 335) detachably enveloped about said block configuration (315).

3. A rack system according to Claim 1, wherein said racks (40; 40'; 40") are releasably coupled to one another to form a plurality of block configurations (315) of said quantity of elongate objects (30; 30'; 30").

4. A rack of the rack system according to any of the preceding Claims, for packaging and processing elongate objects (30; 30'; 30") having an open proximal end (31a; 31'a; 31"a) and a flange (31; 31'; 31") around the object (33; 33'; 33") at said proximal end; the rack (40; 40'; 40") comprising means (48; 80-83) for supporting each of said flanges of said objects; **characterised by**
a base (42; 42'; 42") for supporting the rack (40; 40'; 40") on a surface,
at least two elongate vertical supports (44; 44'; 44"), each having one end connected to said base and connected along its length to said means (48; 80-83) for supporting each of said flanges (31; 31'; 31"); and
a plurality of clips (60; 60'; 60") for releasably retaining in a vertical orientation the bodies (33; 33'; 33") of said objects, said clips being supported by said at least two vertical supports (44; 44'; 44").

5. The rack of Claim 4, wherein said means for supporting each of said flanges comprises:
a top plate (46; 46') and a bottom plate (48; 48') spaced apart from one another to form a pocket for receiving said flanges (31; 31'), a plurality of dividers (52) disposed between said top and bottom plates to divide said pocket into a plurality of individual compartments (53) sized to receive a respective flange, said top plate (46; 46') including a plurality of cut-outs (46a) each disposed over the open proximal end (31a; 31a') of a respective object (33; 33') and said bottom plate (48; 48') including a plurality of second cut-outs (48a) sized to accommodate a respective elongate object (33; 33').

6. The rack of Claim 4, wherein said means for supporting each of said flanges comprises:
a top plate (46") having a top surface and a bottom surface;
a plurality of dividers (84) extending from the bottom surface of said top plate to define between said top plate and said dividers a plurality of individual compartments (53") sized to receive a respective flange (31"),
said top plate (46") including a plurality of cut-outs (46"a) each disposed over the open proximal end (31"a) of an object (33"); and
a side plate (80) extending between said vertical supports (44") in orthogonal relation to said top plate (46"), said side plate defining a plurality of slits (82, 83) sized to retain a portion of a flange (31") introduced into an individual compartment (53").

7. The rack of Claim 4 wherein said plurality of clips (60) are mounted to a bar (62) horizontally supported by said at least two vertical supports (44), further comprising means (66) for indexing said rack (40) along said processing line (10).

8. The rack of Claim 4 wherein said means for releasably coupling comprises:
one or more pins (100) provided on one of said racks; and
one or more depressions (102) on said one of said racks sized to releasably receive a pin (100) provided on another of said racks.

9. The rack of Claim 4 wherein said means for releasably coupling comprises:
a tongue (112; 122; 130) extending in a horizontal manner along a first side of the base (42) of one of said racks (40); and
a groove (110; 120; 132) extending in a horizontal manner along a second side of the base, said second side opposite from the first side, wherein said groove is dimensioned to receive a tongue provided on the base of another of said racks.

## Patentansprüche

1. Ständersystem zum Verpacken und Behandeln einer Anzahl von länglichen Gegenständen, insbesondere Spritzenzylindern (33; 33'; 33"), entlang einer Behandlungslinie (10; 10'), umfassend eine Mehrzahl von Ständern (40; 40'; 40"), die Einrichtungen (48; 80-83) zum lösbaren Halten eines oder mehrerer dieser länglichen Gegenstände in einer vertikalen Ausrichtung haben; **gekennzeichnet durch** jeden dieser Ständer (40; 40'; 40"), die Einrichtungen (100, 102; 100', 102'; 110, 112; 121, 122; 130, 132; 202) zum lösbaren oder flexiblen Aneinanderkoppeln der Ständer haben, und zwar zwecks Bildung einer Blockkonfiguration (200; 315) dieser Anzahl von von den Ständern gehaltenen länglichen Gegenständen, wobei die Blockkonfiguration (200; 315) der länglichen Gegenstände zur Behandlung als Einheit entlang der Behandlungslinie (10; 10') angepasst ist und die Ständer (40; 40'; 40") zur Entkoppelung voneinander oder zur flexiblen Trennung von der Blockkonfiguration angepasst sind, um eine Mehrzahl von Partien oder eine Linienformation der länglichen Gegenstände zur sequentiellen Behandlung entlang der Behandlungslinie (10; 10') zu bilden.

2. Ständersystem gemäß Anspruch 1, weiters umfassend eine Schutzabdeckung (325; 335), die abnehmbar um die Blockkonfiguration (315) gehüllt ist.

3. Ständersystem gemäß Anspruch 1, wobei die Ständer (40; 40'; 40") lösbar aneinandergekoppelt sind, um aus der Anzahl von länglichen Gegenständen (30; 30'; 30") eine Mehrzahl von Blockkonfigurationen (315) zu bilden.

4. Ständer des Ständersystems gemäß einem der vorhergehenden Ansprüche zum Verpacken und Behandeln von länglichen Gegenständen (30; 30'; 30") mit einem offenen proximalen Ende (31a; 31'a; 31"a) und einem Flansch (31; 31'; 31") um den Gegenstand (33; 33'; 33") an diesem proximalen Ende; wobei der Ständer (40; 40'; 40") Einrichtungen (48; 80-83) zum Tragen jedes Flansches mit den Gegenständen umfasst; **gekennzeichnet durch**
einen Untersatz (42; 42'; 42") zum Halten des Ständers (40; 40'; 40") auf einer Oberfläche,
zumindest zwei längliche vertikale Stützen (44; 44'; 44"), von denen jeweils ein Ende mit dem Untersatz verbunden und entlang seiner Länge mit den Einrichtungen (48; 80-83) zum Tragen jedes Flansches (31; 31'; 31") verbunden ist; und
eine Mehrzahl von Klammem (60; 60'; 60") zum lösbaren Halten der Körper (33; 33'; 33") der Gegenstände in einer vertikalen Ausrichtung, wobei die Klammern von den zumindest zwei vertikalen Stützen (44; 44'; 44") getragen werden.

5. Ständer gemäß Anspruch 4, wobei die Einrichtungen zum Tragen jedes Flansches Folgendes umfassen:
eine obere Platte (46; 46') und eine untere Platte (48; 48'), die zwecks Bildung einer Tasche zur Aufnahme der Flansche (31; 31') in einem Abstand voneinander angeordnet sind, eine Mehrzahl von Teilern (52), die zwecks Teilung der Tasche in eine Mehrzahl von zur Aufnahme eines jeweiligen Flansches zugeschnittenen einzelnen Abteilungen (53) zwischen der oberen und der unteren Platte angeordnet sind, wobei die obere Platte (46; 46') eine Mehrzahl von Ausschnitten (46a) einschließt, von denen jeder über dem offenen proximalen Ende (31a; 31a') eines jeweiligen Gegenstands (33; 33') angeordnet ist, und die untere Platte (48; 48') eine Mehrzahl von zweiten Ausschnitten (48a), die zur Unterbringung eines jeweiligen länglichen Objekts (33; 33') zugeschnitten sind, einschließt.

6. Ständer gemäß Anspruch 4, wobei die Einrichtungen zum Tragen jedes Flansches Folgendes umfassen:
eine obere Platte (46") mit einer oberen Fläche und einer unteren Fläche;
eine Mehrzahl von Teilern (84), die sich von der unteren Fläche der oberen Platte erstrecken, um zwischen der oberen Platte und den Teilern eine Mehrzahl von zur Aufnahme eines jeweiligen Flansches (31") zugeschnittenen einzelnen Abteilungen (53") zu definieren,
wobei die obere Platte (46") eine Mehrzahl von Ausschnitten (46"a) einschließt, von denen jeder über dem offenen proximalen Ende (31"a) eines Gegenstands (33") angeordnet ist; und
sich eine Seitenplatte (80) in orthogonaler Beziehung zur oberen Platte (46") zwischen den vertikalen Stützen (44") erstreckt, wobei die Seitenplatte eine Mehrzahl von Schlitzen (82, 83), die zum Halten eines Teils eines in eine einzelne Abteilung (53") eingeführten Flansches (31") zugeschnitten sind, definiert.

7. Ständer gemäß Anspruch 4, wobei die Mehrzahl von Klammern (60) an einer Stange (62) angebracht ist, die von den zumindest zwei vertikalen Stützen (44) horizontal getragen wird, weiters umfassend Einrichtungen (66) zum Umschalten des Ständers (40) entlang der Behandlungslinie (10).

8. Ständer gemäß Anspruch 4, wobei die Einrichtungen zum lösbaren Ankoppeln Folgendes umfassen:
einen oder mehrere Stifte (100), die an einem der Ständer vorgesehen sind; und
eine oder mehrere Vertiefungen (102) an dem einen der Ständer, die zur lösbaren Aufnahme eines an einem anderen Ständer vorgesehenen Stifts (100) zugeschnitten sind.

9. Ständer gemäß Anspruch 4, wobei die Einrichtungen zum lösbaren Ankoppeln Folgendes umfassen:
eine Zunge (112; 122; 130), die sich in horizontaler Weise entlang einer ersten Seite des Untersatzes (42) eines der Ständer (40) erstreckt, und
eine Rille (110; 120; 132), die sich in horizontaler Weise entlang einer zweiten Seite des Untersatzes erstreckt, wobei die zweite Seite der ersten Seite gegenüberliegt und die Rille zur Aufnahme einer am Untersatz eines anderen Ständers vorgesehenen Zunge dimensioniert ist.

## Revendications

1. Système de râtelier pour emballer et traiter une certaine quantité d'objets longs, en particulier des cylindres de seringues (33 ; 33' ; 33'') le long d'une ligne de traitement (10 ; 10') comprenant une pluralité de râteliers (40 ; 40' ; 40'') ayant un dispositif (48 ; 80-83) pour retenir de manière à pouvoir relâcher selon une orientation verticale un ou plusieurs desdits objets longs ; **caractérisé en ce que** chacun desdits râteliers (40 ; 40' ; 40'') ayant un dispositif (100, 102 ; 100' ; 102' ; 110, 112 ; 121, 122 ; 130, 132 ; 202) pour coupler de manière à pouvoir relâcher ou de manière flexible lesdits râteliers l'un à l'autre pour former une configuration de bloc (200 ; 315) de ladite quantité d'objets longs retenus par lesdits râteliers, dans lequel ladite configuration de bloc (200 ; 315) desdits objets longs est adaptée pour être traitée comme une unité le long de ladite ligne de traitement (10 ; 10'), et lesdits râteliers (40 ; 40' ; 40'') sont adaptés pour être découplés l'un par rapport à l'autre ou séparés de manière flexible de ladite configuration de bloc pour former une pluralité de lots ou une formation de ligne desdits objets longs pour le traitement séquentiel le long de ladite ligne de traitement (10 ; 10').

2. Système de râtelier selon la revendication 1, comprenant en outre une enveloppe de protection (325 ; 335) enveloppée de manière détachable autour de ladite configuration de bloc (315).

3. Système de râtelier selon la revendication 1, dans lequel lesdits râteliers (40 ; 40' ; 40'') sont couplés de manière à pouvoir être relâchés l'un de l'autre pour former une pluralité de configurations de bloc (315) de ladite quantité d'objets longs (30 ; 30' ; 30'').

4. Râtelier du système de râtelier selon l'une quelconque des revendications précédentes, pour emballer et traiter des objets longs (30 ; 30' ; 30'') ayant une extrémité proximale ouverte (31a ; 31'a ; 31''a) et un flanc (31 ; 31' ; 31'') autour de l'objet (33 ; 33' ; 33'') au niveau de ladite extrémité proximale ; le râtelier (40 ; 40' ; 40'') comprenant un dispositif (48 ; 80-83) pour supporter chacun desdits flancs desdits objets ; **caractérisé par**
une base (42 ; 42' ; 42'') pour supporter le râtelier (40 ; 40' ; 40'') sur une surface,
au moins deux supports verticaux longs (44 ; 44' ; 44''), chacun ayant une extrémité connectée à ladite base et connectée le long de sa longueur audit dispositif (48 ; 80-83) pour supporter chacun desdits flancs (31 ; 31' ; 31'') ; et
une pluralité de pinces (60 ; 60' ; 60'') pour retenir de manière à pouvoir relâcher selon une orientation verticale les corps (33 ; 33' ; 33'') desdits objets, lesdites pinces étant supportées par lesdits au moins deux supports verticaux (44 ; 44' ; 44'').

5. Râtelier selon la revendication 4, dans lequel ledit dispositif pour supporter chacun desdits flancs comprend :
une plaque supérieure (46 ; 46') et une plaque inférieure (48 ; 48') espacées l'une de l'autre pour former une poche pour recevoir lesdits flancs (31 ; 31'), une pluralité de cloisons (52) disposées entre lesdites plaques supérieure et inférieure pour diviser ladite poche en une pluralité de compartiments individuels (53) calibrés pour recevoir un flanc respectif, ladite plaque supérieure (46 ; 46') comprenant une pluralité d'échancrures (46a), chacune disposée sur l'extrémité proximale ouverte (31a ; 31a') d'un objet respectif (33 ; 33'), et ladite plaque inférieure (48 ; 48') comprenant une pluralité de secondes échancrures (48a) calibrées pour accommoder un objet long respectif (33 ; 33').

6. Râtelier selon la revendication 4, dans lequel ledit dispositif pour supporter chacun desdits flancs comprend :
une plaque supérieure (46'') ayant une surface supérieure et une surface inférieure ;
une pluralité de cloisons (84) s'étendant à partir de la surface inférieure de ladite plaque supérieure pour définir entre ladite plaque supérieure et lesdites cloisons une pluralité de compartiments individuels (53'') calibrés pour recevoir un flanc respectif (31''),
ladite plaque supérieure (46'') comprenant une pluralité d'échancrures (46''a) chacune disposée sur l'extrémité proximale ouverte (31''a) d'un objet (33'') ; et
une plaque latérale (80) s'étendant entre lesdits supports verticaux (44'') selon une relation orthogonale par rapport à ladite plaque supérieure (46''), ladite plaque latérale définissant une pluralité de fentes (82, 83) calibrées pour retenir une partie d'un flanc (31'') introduit dans un compartiment individuel (53'').

7. Râtelier selon la revendication 4, dans lequel ladite pluralité de pinces (60) est montée sur un banc (62) supporté horizontalement par lesdits au moins deux supports verticaux (44), comprenant en outre un dispositif (66) pour indexer ledit râtelier (40) le long de ladite ligne de traitement (10).

8. Râtelier selon la revendication 4, dans lequel ledit dispositif pour coupler de manière à pouvoir relâcher comprend :
une ou plusieurs pinces (100) fournies sur un desdits râteliers ; et
une ou plusieurs dépressions (102) sur un desdits râteliers calibrée(s) pour recevoir de manière à pouvoir relâcher une pince (100) fournie sur un autre desdits râteliers.

9. Râtelier selon la revendication 4, dans lequel ledit dispositif pour coupler de manière à pouvoir relâcher comprend :
une langue (112 ; 122 ; 130) s'étendant de manière horizontale le long d'un premier côté de la base (42) de l'un desdits râteliers (40) ; et
une cannelure (110 ; 120 ; 132) s'étendant de manière horizontale le long d'un second côté de la base, ledit second côté opposé au premier côté, dans laquelle ladite cannelure est dimensionnée de manière à recevoir une langue fournie sur la base d'un autre desdits râteliers.
